# EUROPEAN PATENT SPECIFICATION

(11) **EP 2 892 569 B1**
(45) Date of publication and mention of the grant of the patent: **12.12.2018**
(21) Application number: 13836139.9
(22) Date of filing: 03.09.2013
(51) Int. Cl.: A61K 49/00

(54) **LUMINAL ADMINISTRATION OF TAG MOLECULES FOR DIAGNOSTIC APPLICATIONS**
LUMINALE VERABREICHUNG VON TAG-MOLEKÜLEN FÜR DIAGNOSTISCHE ANWENDUNGEN
ADMINISTRATION LUMINALE DE MOLÉCULES DE MARQUAGE POUR DES APPLICATIONS DE DIAGNOSTIC

(30) Priority: 04.09.2012 US 201261696380 P; 25.01.2013 US 201361756697 P
(43) Date of publication of application: 15.07.2015
(73) Proprietor: Given Imaging Ltd., 20692 Yoqneam (IL)
(72) Inventor: SELLA-TAVOR, Osnat, 19330 Kfar Kish (IL); RABINOVITZ, Elisha, 34992 Haifa (IL)
(74) Representative: HGF Limited
(86) International application number: PCT/IL2013/050747
(87) International publication number: WO 2014/037941

(56) References cited:
- WO-A1-2011/116142
- WO-A1-2012/032524
- WO-A2-2011/086548
- US-A1- 2010 003 196
- US-A1- 2011 262 354
- COHEN ET AL.: 'Engineering of near IR fluorescent albumin nanoparticles for in vivo detection of colon cancer' JOURNAL OF NANOBIOTECHNOLOGY vol. 10, 14 August 2012, pages 1 - 8, XP021107479
- YANG ET AL.: 'Near IR Heptamethine Cyanine Dye-Mediated Cancer Imaging' CLINICAL CANCER RESEARCH vol. 16, 21 April 2010, pages 2833 - 2844, XP055149538
- BLOCH ET AL.: 'The relative roles of charge and a recognition peptide in luminal targeting of colorectal cancer by fluorescent polyacrylamide' EUROPEAN JOURNAL OF PHARMACEUTICAL SCIENCES vol. 47, 25 September 2012, pages 904 - 913, XP055183094

## Description

### FIELD OF THE INVENTION

The invention relates to formulations and methods of luminal administration of tag molecules for diagnostic applications.

### BACKGROUND OF THE INVENTION

It is well known that early diagnosis of cancerous tissue is crucial for increasing the chances of a patient to overcome his disease. Therefore, periodical screening of the population for various types of cancers is important. Nowadays, people above the age of 50 are encouraged by their physician to undergo endoscopy procedures every couple of years, in order to ensure that they do not suffer from colon cancer. However, although people are aware of the importance of early detection of cancer, most of them are reluctant to undergo such invasive and somewhat uncomfortable procedures.

Colonic polyps are slow-growing overgrowths of the colonic mucosa, highly prevalent in the general population, especially with increasing age. While approximately 90% of the polyps are less than 1 cm in diameter and have a low potential for malignancy, the remaining 10% of adenomatous polyps especially those that are larger than 1 cm in diameter, and those of which containing a substantial (>25%) villous component or having high-grade dysplasia commonly carry an increased cancer risk.

Efficient marking of those adenomas by optical probes may significantly improve the detection of cancer.

Some methods of early screening are available. For example, swallowing an autonomous capsule endoscope imaging device, such as the PillCam®, produced by Given Imaging Ltd., Yoqneam, Israel. The capsule endoscope moves along the gastrointestinal tract by natural peristalsis, acquiring images of the lumen as it passes through it. However, in some cases, as may well happen when using standard endoscopes, it may be difficult to identify pathological areas along the lumen, as pathologies may not stand out much compared to the lumen's background.

Therefore, methods of "highlighting" the pathologies compared to the lumen's background have developed. For example, dying the cancerous and/ or the adenoma tissue lumen with near infrared color may assist in easily distinguishing between the cancerous tissue and the noncancerous tissue surrounding it. Near infrared imaging of internal tissues is typically being used, since near infrared radiation exhibits penetration of up to 8 centimeters into the irradiated tissue. This may provide information concerning tumors and polyps that are exposed to the lumen but do not necessarily protrude much out of the lumen wall.

International Patent Application Publication Number WO2011 086548 discloses using a conjugate of a specific polymer and a near infrared dye such as Cy5, Cy5.5 Indocyanine green (ICG), IR783, and analogs thereof. The polymer with the near infrared dye is shown to be inserted into diseased tissue by various means.

"Near IR Heptamethine Cyanine Dye-Mediate Cancer Imaging", 2010, American Association for Cancer Research, by Xiaojian Yang et al., and "A near-infrared fluorescent heptamethine indocyanine dye with preferential tumor accumulation for in vivo imaging", Biomaterials 31 (2010), by Chao Zhang et al., disclose the accumulation of near infrared dye IR783 in human cancer cells, and the dye's retention in those cells and not in normal cells. The human cancer cells were implanted into nude mice, and the near infrared dye was injected into the mice.

WO2012032524 discloses particles comprising either a water-soluble polymer or a phospholipid, wherein at least one near infrared (NIR) fluorescent probe and optionally at least one active agent such as a targeting moiety, capable of selectively recognizing a particular cellular marker, are non-covalently bound to the outer surface of the particles. Pharmaceutical compositions comprising these particles may be used, inter alia, for detection and treatment of tumors in the gastrointestinal tract

Therefore, for improved compliance by healthy population, specific distribution in the gastrointestinal system and less expected adverse effects, there is a need for methods and formulations for luminal administration of the infrared dye for in-vivo detection of cancer in the gastrointestinal system, such as, for example, colon cancer, adenomatous colorectal polyps, stomach cancer or small bowel cancer.

### SUMMARY OF THE INVENTION

Viewed from one aspect, there is provided a tag molecule for use in detecting or imaging pre-cancerous cells and/or adenoma and/or cancer in a gastrointestinal system of a subject as set forth in claim 1 of the appended claims

The present invention relates to tag molecules which may be luminally administered for diagnostic applications. For purposes of screening the population for detection of cancer (e.g., colon cancer, stomach cancer and/or small bowel cancer), there is a need for a simple and user-friendly method of in-vivo insertion of the near infrared dyes other than injection of the dye. Therefore, the present invention discloses a near infrared dye, which may be, for example, IR-783, for luminal administration, i.e., either through oral administration by swallowing a formulation comprising the dye, or through an enema. These two administration methods are simple and comfortable for the patient to undergo and would increase patient compliance to cancer screening methods.

In some embodiments of the present invention, the near infrared dye (e.g., IR-783) may be part of a delivery composition or formulation that comprises a coating (e.g., an enteric coating) or other means of keeping the dye intact prior to reaching the target organ, which is to be tagged. Such delivery compositions are specifically required when the near infrared dye is to be inserted orally.

Cancerous tissue may be tagged by administrating the tag molecule into a patient's gastrointestinal lumen either orally or through an enema, and tagging cancerous tissue present within the lumen with the tag molecule. According to some embodiments, the tag molecule may be a near infrared dye, for example, without limitation, IR-783.

The tagged cancerous tissue may subsequently be imaged. Imaging the tagged cancerous tissue may be conducted by a swallowable imaging capsule. Alternatively, the imaging of the tagged cancerous tissue may be conducted by an endoscope.

The cancerous tissue may be selected from a group consisting of colon cancer, stomach cancer, small bowel cancer, or a combination thereof.

In some embodiments, the invention provides an enteric coated formulation comprising a near infrared dye and a pharmaceutically acceptable carrier. In some embodiments, the coating of the enteric coated formulation comprises methacrylic acid copolymer, which may be Eudragit®L, Eudragit® S, Eudragit® RS, Eudragit® RL, Eudragit® FS 30P, and Eudragit® NE, or a cellulose derivative or any combination thereof.

In some embodiments, the near infrared dye is a fluorescent probe which is IR-783, a derivative of IR-783, e.g., IR-783-S-Ph-COOH, IR-780 iodide, IR-786 iodide, rhodamine 123, a dye such as indocyanine green (ICG), Cy5, Cy5.5, Cy5.18, Cy7 and Cy7.18, IRDye 78, IRDye 680, IRDye 750, IRDye 800 phosphoramidite, DY-681, DY-731, DY-781, Alexa Fluor 610, Alexa Fluor 633, Alexa Fluor 647, Alexa Fluor 660, Alexa Fluor 680, Alexa Fluor 700 and Alexa Fluor 750.

In some embodiments, the formulation may be in the form of a tablet or a capsule. In some embodiments, there is provided a formulation comprising a near infrared dye in a form for being administered in an enema.

The tag molecule may be used for detecting or imaging adenoma and/or cancer or pre-cancerous cells in a gastrointestinal system of a subject. This may be done by: administering to said subject, either orally or through an enema, an effective amount of a formulation comprising a near infrared dye as defined above, and detecting or imaging cells in the gastrointestinal tract lumen that take up said near infrared dye, to determine if cancer is present in the gastrointestinal system of the subject.

There may be a further a step of removing unbound or unspecifically bound near infrared dye.

The cancer to be detected may be colon cancer, adenomatous colorectal polyps, stomach cancer or small bowel cancer. The detecting step may be conducted by an endoscope or by swallowing an autonomous capsule endoscope imaging device. The autonomous capsule endoscope imaging device may be the PillCam®.

The tag molecule may be applied by: administering to the patient, who is on a liquid diet at least a day prior to beginning of the procedure, either orally or through an enema, the formulation defined above, administering a laxative, administering the autonomous capsule endoscope imaging device, and administering a clear liquid agent.

### BRIEF DESCRIPTION OF THE DRAWINGS OF THE INVENTION

The principles and operation of the present invention may be better understood with reference to the drawings, and the following description, it being understood that these drawings are given for illustrative purposes only and are not meant to be limiting, wherein:
FIG. 1 is a graph illustrating the concentration of IR-783 in tumors compared to normal tissue, 24 hours after dye administration in whole body in vivo imaging, as disclosed in prior art.
FIG. 2 is a flow chart of a method of tagging cancerous tissue for diagnostic applications with an embodiment of the invention.
FIG. 3 is a flow chart of a method of tagging cancerous tissue for diagnostic applications with an embodiment of the invention;
FIG. 4 is a flow chart of a method for detecting or imaging cancer in a gastrointestinal system of a subject, using an embodiment of the invention;
FIG. 5A shows a photograph of a colon from an orthotopic mouse with human colorectal tumors;
FIG. 5B shows a photograph of the mouse's colon after 4µg of the IR-783 derivative (IR-783-S-Ph-COOH) were introduced into the colon of the mouse via a minicolonoscope inserted through the mouse's rectum;
FIGS. 6A-B show photographs of colons of orthotopic mice with human colorectal tumors; FIG. 6A shows intensely labeled colon tumors, and FIG. 6B shows non-labeled colon tumors; and
FIG. 7 shows fluorescent photographs of the liver, spleen, heart, lungs, kidneys and brain of mice administered with 4µg of IR-783 by gastric gavage.

### DETAILED DESCRIPTION OF THE INVENTION

In the following detailed description, numerous specific details are set forth in order to provide a thorough understanding of the invention. However, it will be understood by those of ordinary skill in the art that the present invention may be practiced without these specific details. In other instances, well-known methods, procedures, components and circuits have not been described in detail so as not to obscure the present invention.

The invention describes formulations of tag molecules for diagnostic applications. For purposes of screening the population for detection of cancer (e.g., colon cancer, stomach cancer and/or small bowel cancer), there is a need for a simple and user-friendly method of in-vivo insertion of the near infrared dyes other than injection of the dye.

In one embodiment of the invention, there is provided an enteric coated formulation comprising a near infrared dye and a pharmaceutically acceptable carrier. The near infrared dye, which is also referred to herein as the tag molecule, may be a fluorescent probe which is IR-783, a derivative of IR-783, IR-780 iodide, IR-786 iodide, rhodamine 123, a dye such as indocyanine green (ICG), Cy5, Cy5.5, Cy5.18, Cy7 and Cy7.18, IRDye 78, IRDye 680, IRDye 750, IRDye 800 phosphoramidite, DY-681, DY-731, DY-781, Alexa Fluor 610, Alexa Fluor 633, Alexa Fluor 647, Alexa Fluor 660, Alexa Fluor 680, Alexa Fluor 700 and Alexa Fluor 750, or any combination thereof.

In some embodiments of the invention, the near infrared dye is IR-783.

In some embodiments of the invention, the derivative of IR-783 is IR-783-S-Ph-COOH.

According to some embodiments, the coating of the enteric coated formulation of the invention comprises methacrylic acid copolymer. The methacrylic acid copolymer may be one or more of Eudragit®L, Eudragit® S, Eudragit® RS, Eudragit® RL, Eudragit® FS 30P, and Eudragit® NE, or a cellulose derivative.

In some embodiments, there is provided a formulation comprising a near infrared dye in a form suitable for being administered in an enema. The near infrared dye may be a fluorescent probe which is IR-783, a derivative of IR-783, IR-780 iodide, IR-786 iodide, rhodamine 123, a dye such as indocyanine green (ICG), Cy5, Cy5.5, Cy5.18, Cy7 and Cy7.18, IRDye 78, IRDye 680, IRDye 750, IRDye 800 phosphoramidite, DY-681, DY-731, DY-781, Alexa Fluor 610, Alexa Fluor 633, Alexa Fluor 647, Alexa Fluor 660, Alexa Fluor 680, Alexa Fluor 700 and Alexa Fluor 750, or any combination thereof.

In some embodiments of the invention, the near infrared dye is IR-783.

The oral pharmaceutical compositions of the invention may also be formulated as a controlled-release matrix. For example, controlled-release matrix tablets in which the release of a soluble active ingredient is controlled by having the active ingredient diffuse through a gel formed after the swelling of a hydrophilic polymer brought into contact with a dissolving liquid (when used in vitro) or gastro-intestinal fluid (when used in vivo). Many polymers have been described as capable of forming such gel, e.g., derivatives of cellulose, cellulose ethers such as hydroxypropyl cellulose, hydroxymethyl cellulose, methylcellulose or hydroxypropyl methyl cellulose, or ethers having fairly high viscosity. According to other embodiments, the compositions comprise the active ingredient formulated for controlled release in a microencapsulated dosage form, in which small droplets of the active ingredient are surrounded by a coating or a membrane to form particles in the range of a few micrometers to a few millimeters.

Some embodiments for preparing the formulation are depot systems, which are based on biodegradable polymers, wherein as the polymer degrades the active ingredient is slowly released. Typically, hydrolytically labile polyesters prepared from lactic acid, glycolic acid, or combinations thereof may be used. Examples for biodegradable polymers prepared from these particular monomers include, without being limited to, poly(D,L-lactide) (PLA), polyglycolide (polyglycolic acid; PGA), and the copolymer poly(D,L-lactide-co-glycolide) (PLGA).

In some embodiment, the oral formulation may be in a form of a tablet. The tablet may comprise at least one filler, e.g., lactose, ethylcellulose, microcrystalline cellulose, silicified microcrystalline cellulose; at least one disintegrant, e.g., cross-linked polyvinylpyrrolidinone; at least one binder, e.g., polyvinylpyridone, hydroxypropylmethyl cellulose; at least one surfactant, e.g., sodium laurylsulfate; at least one glidant, e.g., colloidal silicon dioxide; and at least one lubricant, e.g., magnesium stearate.

In some embodiments, the oral formulation is in a form of a capsule.

In certain embodiments, the pharmaceutical composition or formulation of the invention, when formulated for oral administration, is in the form of a monolithic matrix, i.e., a structure including a three-dimensionally stable matrix material having a discrete size and shape; a tablet such as a bi-layered or multilayered tablet, matrix tablet, disintegrating tablet, dissolving tablet, or chewable tablet; or a capsule or sachet, e.g., filled with granules, grains, beads, or pellets.

In some embodiments of the invention, the amount of the near infrared dye is between 10 mg and 1000 mg. In some embodiments, the amount of the near infrared dye is between 10-200 mg. In some embodiments, the amount is about 10, 20, 30, 40, 50, 60, 70, 80, 90, 100, 110, 120, 130, 140, 150, 160, 170, 180, 190 or 200 mg ± 10%. It is noted that in some embodiments, the formulation may be administered to the patient more than one time prior to the detection procedure.

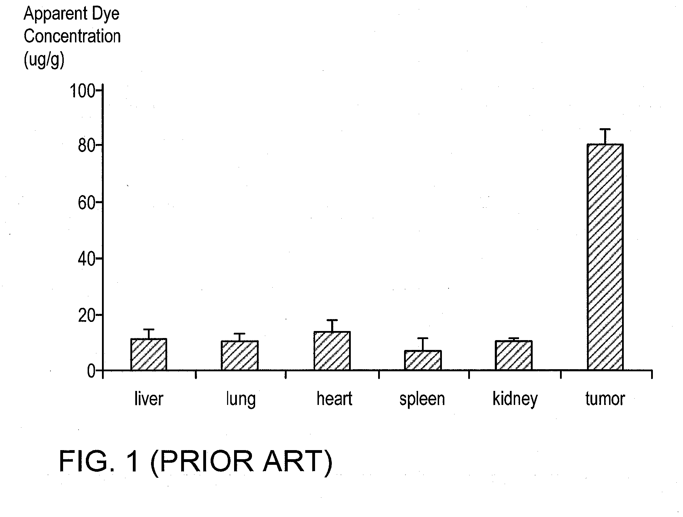

Reference is now made to FIG. 1, which is a graph illustrating the concentration of near infrared IR-783 dye in tumors compared to normal tissue, 24 hours after dye administration, by injection, in whole body in vivo imaging, as disclosed in the prior art. FIG. 1 shows that the IR-783 dye concentration is much higher in tumors compared to the dye concentration in normal body tissue. This proves that IR-783 may be efficiently used for tagging cancerous tissue and enabling sufficient differentiation between cancerous tissue and normal tissue surrounding it.

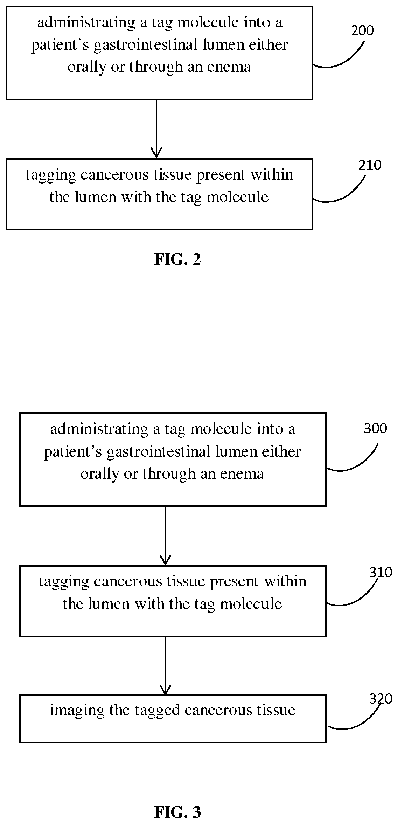

Reference is now made to FIG. 2, which is a flow chart of a method of tagging cancerous tissue for diagnostic applications with an embodiment of the invention. The method may comprise the following steps of: administrating the formulation of the invention into a patient's gastrointestinal lumen either orally or through an enema (200), and tagging cancerous tissue present within the lumen with the tag molecule (210).

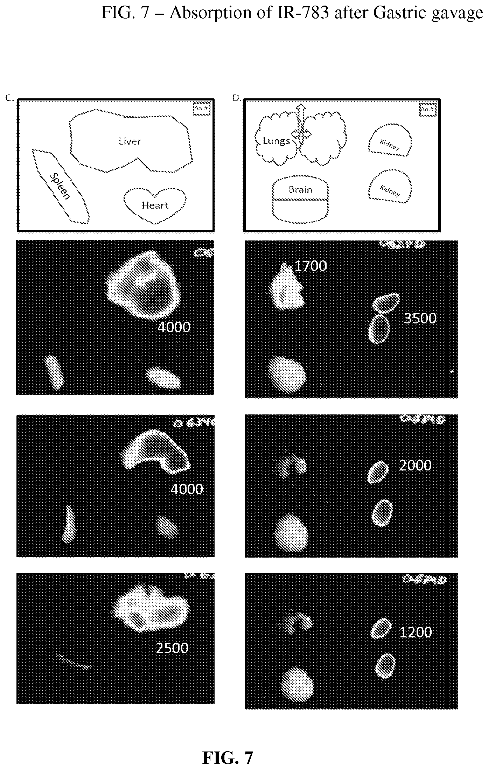

In some embodiments of the invention, the near infrared dye may be part of a delivery composition or formulation that comprises a coating or other means of keeping the dye intact prior to reaching the target organ. As explained in Example 3 and as shown in FIG. 7, part of the dye that was orally administered was absorbed in the stomach and thus did not reach the colon lumen. Therefore, formulations that protect the dye in the stomach for further release of it in the colon are required.

For example, the near infrared dye may be coated with enteric coating so that the dye may pass the acidic fluids of the stomach, while staying intact, until the dye reaches the small bowel or colon. Such delivery compositions are specifically required when the near infrared dye is to be inserted orally.

The step of tagging the cancerous tissue with the tag molecule may assist in diagnosing cancer within the lumen. The tissue tagged with a near infrared dye may be imaged with near infrared imaging devices, e.g., capsule endoscopes, standard endoscopes or other devices that incorporate near infrared imaging means. Since only the cancerous tissue is tagged with the tag molecule (e.g., IR-783), see Examples 1 and 2, it may be apparent from the images which section of the tissue is the cancerous tissue and which is normal tissue. A swallowable in-vivo device/capsule, such as an autonomous swallowable capsule, may be used for imaging the tagged tissue. Alternatively, the in-vivo device need not be swallowable or autonomous, and may have other shapes or configurations, e.g., a standard endoscope, though other imaging devices may be used.

The tissue that is to be tagged by the tag molecule (e.g., IR-783), may suffer from colon cancer, stomach cancer, small bowel cancer, or a combination thereof.

Reference is now made to Fig. 3, which is a flow chart of a method of tagging cancerous tissue for diagnostic applications. The method according to FIG. 3, may comprise the steps of: administrating the formulation of the invention into a patient's gastrointestinal lumen either orally or through an enema (300), tagging cancerous and or adenoma tissue present within the lumen with the tag molecule (310), and imaging the tagged cancerous tissue (320). The step of imaging the tagged cancerous tissue may be done by in-vivo devices such as capsule endoscopes, endoscopes or any other in-vivo device that may incorporate near infrared imaging means.

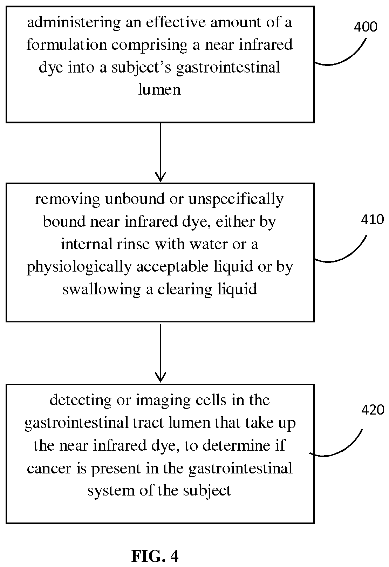

Reference is now made to FIG. 4, which is a flow chart of a method for detecting or imaging cancer in a gastrointestinal system of a subject. The method may comprise the steps of administering to said subject's gastrointestinal tract an effective amount of a formulation comprising a near infrared dye according to embodiments of the invention (400); and detecting or imaging cells in the gastrointestinal tract lumen that take up said near infrared dye, to determine if cancer is present in the gastrointestinal system of the subject (420). The method may comprise a step of removing unbound or unspecifically bound near infrared dye, either by internal rinse with water or a physiologically acceptable liquid or by swallowing a clearing liquid (410). The method may enable detecting or imaging precancerous cells in a gastrointestinal system of a subject. The method may comprise the steps of: (a) administering to said subject an effective amount of a formulation comprising a near infrared dye according to an embodiment of the invention; and (b) detecting or imaging pre -cancerous cells that absorb or interact with the near infrared dye.

Prior to administration of the pharmaceutical composition, the gastrointestinal tract of the treated subject may be purged using appropriate fluids, and following administration, suitable fluids may further be administered to rinse non-attached particles so as to ensure that near infra-red (NIR) emission is detected essentially from regions having particles bound through their targeting moieties only, i.e., regions containing cancer or a pre-cancer tissues.

The method may involves a liquid diet for a day or more prior to beginning the procedure, i.e., before the step of administering the formulation of the invention. A laxative may be administered, before, after or simultaneously with the step of administering the formulation of the invention.

A method for detecting or imaging cancerous or pre-cancer cells in the gastrointestinal tract may comprise administering to the patient, who is on a liquid diet for at least a day prior to the beginning of procedure, the formulation of the invention; administering a laxative, before, after or simultaneously with administering the formulation; and administering an autonomous capsule endoscope imaging device. The method may further comprise the step of administering a clear liquid agent following the step of administering the autonomous capsule endoscope.

The patient may, for example, begin a liquid diet 24hr prior to the procedure. On the evening of the first day, the patient is administered with the formulation of the invention, along with a laxative. The laxative may be administered before or after the formulation is administered. In the morning of the second day (i.e., the day of the procedure), the patient is administered with an autonomous capsule endoscope imaging device. The patient may be administered with a booster laxative (to increase bowel activity), a few hours (e.g., between about 2 to 10hr) after the administration of the autonomous capsule endoscope imaging device.

Near infrared emission may be detected from the walls of the gastrointestinal tract by any suitable means, e.g., by means of colonoscopy or endoscopic capsules, a tube with an optical fiber, a swallowed capsule with a detector that transmits information to a receiver, or by an NIR detector that is placed outside the body.

The disclosed methods may not require processing of images; rather, in one variation, a surgeon or clinician, through the use of, e.g., intravascular probe or an endoscope, can quickly scan areas of suspected tumor growth and use the level of fluorescence to more precisely discriminate tumor tissue from non-tumor tissue and thereby more precisely define tumor borders for surgical resection or diagnostic evaluation, or for laser or radiation therapy, including brachytherapy and external beam therapy, or for improved biopsy procedures. In other variations, processing of images acquired by the imaging device may either be performed by a processor external or internal to the imaging device.

The method may be repeated one or more times so as to enable monitoring of the therapeutic effects of a medicament. The tumors in the patient's luminal tract may be detected by methods described hereinabove. The patient may then receive the appropriate medicament to treat the detected tumors. After a certain period of time, the patient's luminal tract may be monitored so as to assess whether the tumors' size was reduced or diminished.

There may be provided a kit with the oral formulation (or pharmaceutical composition) of the invention or with the formulation for an enema, or the combination thereof. The kit may further comprise a laxative. The kit may further comprise a clear liquid agent or a booster laxative. The kit may further comprise a laxative and a clear liquid agent or a booster laxative. There may be provided a kit with the oral formulation of the invention or with the formulation for an enema, or the combination thereof, a laxative and a clear liquid agent (or a booster laxative) and instructions for the use and the regimen of each of the ingredients.

While certain features of the present invention have been illustrated and described herein, many modifications, substitutions, changes, and equivalents may occur to those of ordinary skill in the art.

### Examples

### Example 1

Orthotopic mice with human colorectal tumors in their colons were used for gastric gavage administration of the IR-783 derivative - IR-783-S-Ph-COOH. 4µg of the IR-783-S-Ph-COOH dissolved in 200µl PBS were introduced into the colon of the mouse via minicolonoscope inserted through the rectum. The IR-783-S-Ph-COOH solution was incubated in the colon for 20 minutes and then washed out with 5 ml PBS inserted through the rectum. Four hours later, mice were sacrificed and the fluorescence of the tumor and the surrounding colon tissue was measured by a fluorescent camera setup. Histopathological examination was performed on paraffin fixed colonic tissue specimens.

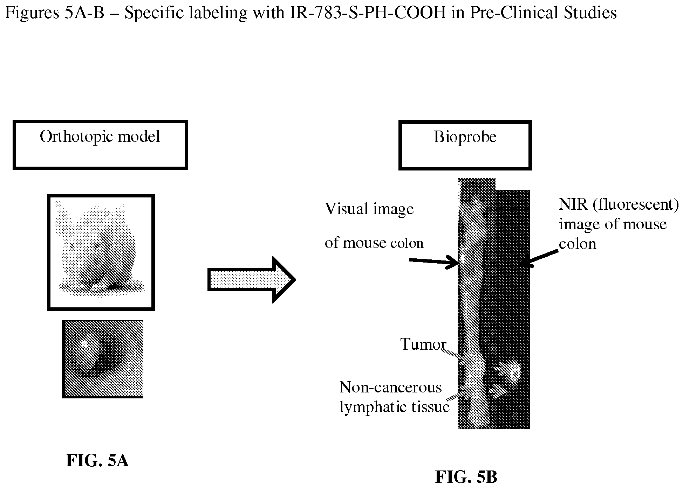

As can be seen from FIG. 5B, two regions suspected as tumors were observed. One of them was intensely labeled, with tumor to background ratio of 60 in 1 second exposure. This region was confirmed as a tumor in histopathologic examination. The second region was not labeled with the fluorescence material, and the histopathological examination confirmed that this region contains only non-cancerous lymphatic tissue with no tumor cells.

### Example 2

Orthotopic mice with human colorectal tumors in their colons were used for luminal administration of IR-783. 4µg of IR-783 dissolved in 200µl PBS were introduced into the colon of the mouse via a minicolonoscope inserted through the rectum. The IR-783 solution was incubated in the colon for 20 minutes and then washed out with 5 ml of PBS. 4h later, the mice were sacrificed and the fluorescence of the tumor and the surrounding colon was measured by a fluorescent camera setup. Histopathological examination was performed on paraffin fixed colonic tissue specimens.

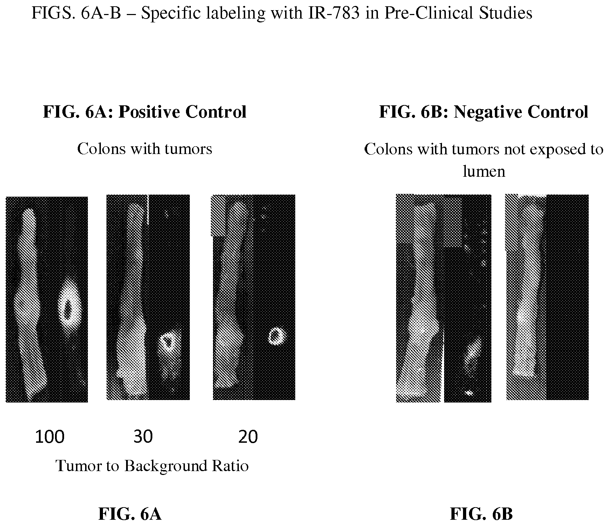

In FIG. 6A tumors intensely labeled are shown. Tumor to background ratios are indicated. Histopathologic examination of the labeled tumors confirmed the presence of tumor that grew over the murine mucosal layer and therefore are exposed to the mice colon lumen. In FIG. 6B tumors that were not labeled are shown. Further histopathology examination of these tumors verified that they were developed below the murine mucosal layer and therefore were not exposed to the colon lumen and could not become in contact with the IR-783 solution. Since colon cancer in humans is always developed over the mucosal layer (and not below the mucosal layer), it may be concluded that colon tumors in the human body may be efficiently and specifically labeled by IR783.

### Example 3

4µg of IR-783 dissolved in 200 µl PBS were introduced into mice stomach by gastric gavage. Five hours later, the mice were sacrificed and liver, spleen, heart, lungs, kidneys and brain were collected and measured for fluorescence by a fluorescent camera setup. As can be seen from FIG. 7, fluorescence was detected in the liver and kidneys. Grey levels of the organs are indicated by the numbers on the fluorescent pictures next to the imaged organs, e.g., grey level in the liver was 4000 in two mice and 2500 in a third mouse, and grey levels in the kidneys were 3500, 200 and 1200, respectively.

These results suggest that part of the dye was absorbed in the stomach and thus did not reach the colon lumen. Therefore, formulations that protect the dye in the stomach for further release in the colon are required. For example, formulations with enteric coating may ensure the formulations stay intact prior to the formulations reaching the target location, e.g., the colon.

## Claims

1. A tag molecule for use in detecting or imaging pre-cancerous cells and/or adenoma and/or cancer in a gastrointestinal system of a subject, wherein the tag molecule is a near infrared dye, wherein the tag molecule is administered into the subject's gastrointestinal lumen either orally or by way of an enema and wherein the pre-cancerous cells and/or adenoma and/or cancer are tagged with the tag molecule within the lumen.

2. The tag molecule of claim 1, wherein the cancer is colon cancer, adenomatous colorectal polyps, stomach cancer or small bowel cancer.

3. The tag molecule of any preceding claim, in the form of an enteric coated formulation comprising the near infrared dye and a pharmaceutically acceptable carrier.

4. The tag molecule of claim 3, wherein the coating of said enteric coated formulation comprises methacrylic acid copolymer.

5. The tag molecule of claim 4, wherein said methacrylic acid copolymer is Eudragit®L, Eudragit® S, Eudragit® RS, Eudragit® RL, Eudragit® FS 30P, and Eudragit® NE, or a cellulose derivative or any combination thereof.

6. The tag molecule of claim 1, wherein the near infrared dye is a fluorescent probe which is IR-783, a derivative of IR-783, IR-780 iodide, IR-786 iodide, rhodamine 123, a dye such as indocyanine green (ICG), Cy5, Cy5.5, Cy5.18, Cy7 and Cy7.18, IRDye 78, IRDye 680, IRDye 750, IRDye 800 phosphoramidite, DY-681, DY-731, DY-781 Alexa Fluor 61 0, Alexa Fluor 633, Alexa Fluor 647, Alexa Fluor 660, Alexa Fluor 680, Alexa Fluor 700 and Alexa Fluor 750.

7. The tag molecule of claim 1, wherein the near infrared dye is IR-783.

8. The tag molecule of claim 7, wherein the IR-783 derivative is IR-783-S-Ph-COOH.

9. The tag molecule of claim 1, in the form of a tablet, a capsule or an enema.

## Patentansprüche

1. Tag-Molekül zur Verwendung bei der Erkennung oder Bildgebung von präkanzerösen Zellen und/oder Adenomen und/oder Krebs in einem Gastrointestinaltrakt eines Individuums, wobei das Tag-Molekül ein Nahinfrarotfarbstoff ist, wobei das Tag-Molekül in das gastrointestinale Lumen des Individuums entweder oral oder mittels eines Einlaufs verabreicht wird und wobei die präkanzerösen Zellen und/oder das Adenom und/oder der Krebs mit dem Tag-Molekül innerhalb des Lumens markiert werden.

2. Tag-Molekül nach Anspruch 1, wobei der Krebs Kolonkrebs, adenomatöse Kolorektalpolypen, Magenkrebs oder Dünndarmkrebs ist.

3. Tag-Molekül nach einem der vorstehenden Ansprüche in Form einer magensaftresistenten Formulierung, die den Nahinfrarotfarbstoff und einen pharmazeutisch unbedenklichen Träger umfasst.

4. Tag-Molekül nach Anspruch 3, wobei die Beschichtung der magensaftresistenten Formulierung ein Methacrylsäure-Copolymer umfasst.

5. Tag-Molekül nach Anspruch 4, wobei das Methacrylsäure-Copolymer Eudragit® L, Eudragit® S, Eudragit® RS, Eudragit® RL, Eudragit® FS 30P und Eudragit® NE oder ein Cellulosederivat oder eine Kombination davon ist.

6. Tag-Molekül nach Anspruch 1, wobei der Nahinfrarotfarbstoff eine fluoreszierende Sonde ist, die IR-783, ein Derivat von IR-783, IR-780-Jodid, IR-786-Jodid, Rhodamin 123, ein Farbstoff wie Indocyangrün (ICG), Cy5, Cy5.5, Cy5.18, Cy7 und Cy7.18, IRDye 78, IRDye 680, IRDye 750, IRDye 800 Phosphoramidit, DY-681, DY-731, DY-781 Alexa Fluor 61 0, Alexa Fluor 633, Alexa Fluor 647, Alexa Fluor 660, Alexa Fluor 680, Alexa Fluor 700 und Alexa Fluor 750 ist.

7. Tag-Molekül nach Anspruch 1, wobei der Nahinfrarotfarbstoff IR-783 ist.

8. Tag-Molekül nach Anspruch 7, wobei das IR-783-Derivat IR-783-S-Ph-COOH ist.

9. Tag-Molekül nach Anspruch 1 in Form einer Tablette, einer Kapsel oder eines Einlaufs.

## Revendications

1. Molécule de marquage pour utilisation dans la détection ou l'imagerie de cellules précancéreuses et/ou d'un adénome et/ou d'un cancer dans un système gastro-intestinal d'un sujet, ladite molécule de marquage étant un colorant dans l'infrarouge proche, ladite molécule de marquage étant administrée dans la lumière gastro-intestinale du sujet soit par voie orale soit au moyen d'un lavement et lesdites cellules précancéreuses et/ou ledit adénome et/ou ledit cancer étant marqués avec la cellule de marquage à l'intérieur de la lumière.

2. Molécule de marquage selon la revendication 1, ledit cancer étant un cancer du côlon, des polypes adénomateux colorectaux, un cancer de l'estomac ou un cancer de l'intestin grêle.

3. Molécule de marquage selon l'une quelconque des revendications précédentes, sous la forme d'une formulation à enrobage entérique comprenant le colorant dans l'infrarouge proche et un excipient pharmaceutiquement acceptable.

4. Molécule de marquage selon la revendication 3, ledit enrobage de ladite formulation à enrobage entérique comprenant un copolymère d'acide méthacrylique.

5. Molécule de marquage selon la revendication 4, ledit copolymère d'acide méthacrylique étant l'Eudragit® L, l'Eudragit® S, l'Eudragit® RS, l'Eudragit® RL, l'Eudragit® FS 30P et l'Eudragit® NE ou un dérivé de cellulose ou toute combinaison de ceux-ci.

6. Molécule de marquage selon la revendication 1, ledit colorant dans l'infrarouge proche étant une sonde fluorescente qui est l'IR-783, un dérivé de l'IR-783, l'iodure d'IR-780, l'iodure d'IR-786, la rhodamine 123, un colorant tel que le vert d'indocyanine (ICG), la Cy5, la Cy5.5, la Cy5.18, la Cy7 et la Cy7.18, l'IRDye 78, l'IRDye 680, l'IRDye 750, l'IRDye 800 phosphoramidite, le DY-681, le DY-731, le DY-781 l'Alexa Fluor 61 0, l'Alexa Fluor 633, l'Alexa Fluor 647, l'Alexa Fluor 660, l'Alexa Fluor 680, l'Alexa Fluor 700 et l'Alexa Fluor 750.

7. Molécule de marquage selon la revendication 1, ledit colorant dans l'infrarouge proche étant l'IR-783.

8. Molécule de marquage selon la revendication 7, ledit dérivé de l'IR-783 étant l'IR-783-S-Ph-COOH.

9. Molécule de marquage selon la revendication 1, sous la forme d'un comprimé, d'une capsule ou d'un lavement.
